# EUROPEAN PATENT SPECIFICATION

(11) **EP 4 645 228 B1**
(45) Date of publication and mention of the grant of the patent: **01.04.2026**
(21) Application number: 25172171.8
(22) Date of filing: 24.04.2025
(51) Int. Cl.: G06T 11/00, G16H 30/40

(54) **MACHINE LEARNING AIDED REALISM-ENHANCED VIRTUAL CLINICAL IMAGING WITH GROUND TRUTH PRESERVATION**
DURCH MASCHINENLERNEN UNTERSTÜTZTE REALISMUSVERSTÄRKTE VIRTUELLE KLINISCHE BILDGEBUNG MIT GRUNDWAHRHEITSERHALTUNG
IMAGERIE CLINIQUE VIRTUELLE AMÉLIORÉE PAR RÉALISTIQUE ASSISTÉE PAR APPRENTISSAGE AUTOMATIQUE AVEC PRÉSERVATION DE RÉALITÉ DE TERRAIN

(30) Priority: 29.04.2024 US 202418649219
(43) Date of publication of application: 05.11.2025
(73) Proprietor: Siemens Healthineers AG, 91301 Forchheim (DE); Duke University, Durham, North Carolina 27705 (US)
(72) Inventor: SAHBAEE, Pooyan, Bethesda, MD, 20817 (US); SAMEI, Ehsan, Chapel Hill, NC, 27514 (US)
(74) Representative: HKW Intellectual Property PartG mbB

(56) References cited:
- RUSS TOM ET AL: "Synthesis of CT images from digital body phantoms using CycleGAN", INTERNATIONAL JOURNAL OF COMPUTER ASSISTED RADIOLOGY AND SURGERY, SPRINGER, DE, vol. 14, no. 10, 5 August 2019 (2019-08-05), pages 1741 - 1750, XP036908874, ISSN: 1861-6410, [retrieved on 20190805], DOI: 10.1007/S11548-019-02042-9
- KIM GIHUN ET AL: "Power-law spectrum-based objective function to train a generative adversarial network with transfer learning for the synthetic breast CT image", 20231004, vol. 68, no. 20, 4 October 2023 (2023-10-04), XP020488074, DOI: 10.1088/1361-6560/ACFADF
- WANG YUE ET AL: "TWIN-GPT: Digital Twins for Clinical Trials via Large Language Model", ACM TRANSACTIONS ON MULTIMEDIA COMPUTING, COMMUNICATIONS, AND APPLICATIONS, 1 April 2024 (2024-04-01), XP093302136, Retrieved from the Internet <URL:https://arxiv.org/pdf/2404.01273v1>
- YIHAO ZHAO ET AL: "Unpaired Image-to-Image Translation using Adversarial Consistency Loss", ARXIV.ORG, CORNELL UNIVERSITY LIBRARY, 201 OLIN LIBRARY CORNELL UNIVERSITY ITHACA, NY 14853, 18 January 2021 (2021-01-18), XP081860445

## Description

### FIELD

This disclosure relates to the use of Virtual Clinical Trials and Digital Twins for medical imaging.

### BACKGROUND

Virtual Clinical Trials (VCTs) serve as a cornerstone in modern medical research, allowing for the optimization, validation, and evaluation of imaging systems without actual patient exposure. While physical phantoms provide tangible reference points, VCTs offer controlled, efficient, and repeatable experiments. In addition, post-processing models, including AI models, especially those in medical imaging, continue to evolve and require vast amounts of data for training and optimization, there's a rising dependence on synthetic or Virtual Clinical Trial (VCT) data. These methodologies, like VCT, have grown significantly in importance due to their ability to provide a controlled yet diverse set of data. Yet, their effectiveness hinges on data realism and accuracy.

A paper by Tom Russ et al., "Synthesis of CT images from digital body phantoms using CycleGAN", discloses that the potential of medical image analysis with neural networks is limited by the restricted availability of extensive data sets. The incorporation of synthetic training data is one approach to bypass this shortcoming, as synthetic data offer accurate annotations and unlimited data size. The authors evaluated eleven CycleGAN for the synthesis of computed tomography (CT) images based on XCAT body phantoms. The image quality was assessed in terms of anatomical accuracy and realistic noise properties.

A paper by Gihun Kim and Jongduk Baek, " Power-law spectrum-based objective function to train a generative adversarial network with transfer learning for the synthetic breast CT image" proposes a new objective function to improve the quality of synthesized breast CT images generated by a GAN and compares the GAN performances on transfer learning datasets from different image domains. The proposed objective function, named beta loss function, is based on the fact that x-ray-based breast images follow the power-law spectrum. The beta loss function is defined in terms of L1 distance between the beta value of synthetic images and validation samples.

A paper by Yue Wang et al., "TWIN-GPT: Digital Twins for Clinical Trials via Large Language Model", proposes a large language model-based digital twin creation approach, called TWIN-GPT. TWIN-GPT can establish cross-dataset associations of medical information given limited data, generating unique personalized digital twins for different patients, thereby preserving individual patient characteristics.

### SUMMARY

The invention is defined by the attached set of claims. Further details of the disclosed method, devices and system are described below, which are helpful for understanding the claimed invention.

By way of introduction, the preferred embodiments described below include methods, systems, instructions, and computer readable media for machine learning aided realism-enhanced virtual clinical imaging with ground truth preservation.

In a first aspect, a method for training a model for unpaired Image-to-Image translation of medical images, the method comprising: acquiring a plurality of real patient images; acquiring a plurality of synthetic images of digital phantoms; and training a model to transform the plurality of synthetic images of digital phantoms to resemble the plurality of real patient images, wherein the model is trained using at least one specialized loss function that ensures the transformed images retains original values of the plurality of synthetic images.

In a second aspect, a system for performing a virtual clinical trial, the system comprising: a plurality of virtual digital phantoms and/or physical phantoms, wherein each of the virtual digital phantoms and/or physical phantoms includes one or more ground truth values for a feature included in the virtual digital phantoms and/or physical phantoms; a virtual imaging simulator configured to generate a virtual image from each of the plurality of virtual digital phantoms and/or physical phantoms; and a model configured for unpaired Image-to-Image translation, the model configured to transform the virtual images to resemble real patient images while maintaining the one or more ground truth values.

In a third aspect, a method for generating a synthetic medical image, the method comprising: selecting a digital phantom; inputting the digital phantom into a medical imaging simulator configured to generate a simulated image of the digital phantom; inputting the simulated image into an unpaired image to image translation network configured to generate a realistic version of the simulated image; and evaluating the realistic version of the simulated image.

It is understood that the third aspect may deploy a model if the first aspect or a system of the second aspect as an image to image translation network. The first aspect may be used to provide the model for the second aspect.

The present invention is defined by the following claims, and nothing in this section should be taken as a limitation on those claims. Further aspects and advantages of the invention are discussed below in conjunction with the preferred embodiments and may be later claimed independently or in combination.

### BRIEF DESCRIPTION OF THE DRAWINGS

Figure 1 depicts an example of a system for machine learning aided realism-enhanced virtual clinical imaging with ground truth preservation according to an embodiment.
Figure 2 depicts an example method for training a machine learned network for realism-enhanced virtual clinical imaging with ground truth preservation according to an embodiment.
Figure 3 depicts an example CT scanning system.
Figure 4 depicts an example process for generating images from an XCAT phantom.
Figure 5 depicts an example GAN architecture.
Figure 6 depicts an example CycleGAN architecture.
Figure 7 depicts an example StarGAN architecture.
Figure 8 depicts an example workflow for generating an image using realism-enhanced virtual clinical imaging with ground truth preservation according to an embodiment.
Figure 9 depicts an example of the image translation process according to an embodiment.

### DETAILED DESCRIPTION

Embodiments provide systems and methods for machine learning aided realism-enhanced virtual clinical imaging with ground truth preservation. An unpaired image to image network is trained to transfer a realistic style derived from real patient images to simulated images generated using a phantom.

Virtual clinical trials (VCTs) and Virtual imaging trails (VITs) are an efficient alternative to clinical trials for evaluating and optimizing imaging concepts and technologies. In a VCT, the human subject is replaced with a virtual digital phantom, the imaging system with a virtual simulated scanner, and the clinical interpretation with a virtual interpretation. The subject is imaged and the image interpreted emulating the clinical process without an actual clinical trial. Another use for virtual / synthetic images is in creating and analyzing digital twins of physical objects such as 3D printed phantoms. The digital twin may include a connection between the physical object and the digital object to enable bi-directional real-time impact on each other. Alternatively, the digital twin may be static. The digital twin may be used for various medical operations or procedures. For example, the digital twin may be used to simulate surgical approaches before an actual procedure. The digital twin may be used for medical device design. The digital twin may be used in VCTs or VITs.

Different methods may be used to create phantoms including fully digital phantoms and physical 3D printed phantoms. Phantoms are generated by defining objects to represent the necessary organs and structures of a given subject. The anatomical objects are assigned tissue material properties (density, elemental composition, radioactivity uptake, magnetic resonance, acoustical properties, etc.) for input into corresponding imaging simulations including, for example, x-ray, computed tomography (CT), nuclear medicine, magnetic resonance imaging (MRI), and ultrasound. The imaging simulator generates image(s) from the phantoms.

Certain patient-based phantoms may be derived directly from human subject images. Since each phantom is recreated from a real human body (or portion thereof), the appearance is inherently realistic, including distributions that cannot be readily reproduced by procedural techniques. However, this patient-based approach has some key limitations: first each subject yields one phantom, so the number and diversity of phantoms are limited by finite human subject data, second the process of generating phantoms may be computationally expensive, and third source data come from medical images, which have limitations of contrast, resolution, noise, and artifacts and may in turn affect the quality of the phantom, typically by limiting its resolution.

Computational phantoms are not derived directly from human subject images but rather crafted, by, for example, hand or using one or more algorithms. One advantage of computational phantoms is that, unlike actual patients, their exact anatomy is known, providing a "gold standard" or "ground truth" from which to quantitatively evaluate and improve imaging devices and techniques. Imaging data of a computational phantom may be generated using a computerized scanner model under various scanning parameters or protocols, and the effects quantified in comparison with the known phantom. The user knows precisely what simulated images should reveal in terms of organ volumes or boundaries, tumor locations, sizes, shapes, extent and frequency of motion, presence, and location of disease indicators, etc. The dose to the organs and structures from different procedures may also be calculated to assess patient risk from radiation exposure. None of these things is possible using live subjects. However, one fundamental issue is that while images from computational phantoms such as extended cardiac-torso (XCAT) images, present accurate ground truth, they don't convincingly reproduce the realism and variability observed in patient scans.

One way to provide realism and variability is by using image-to-image translation. Image-to-image translation is an image generation task that converts source images to a target domain with a particular style while maintaining the content from the source domain. Unpaired image-to-image translation refers to the method of translating an image from a source domain to a target domain without paired training data. The goal is to provide a translation result that is constructed of content from the source domain and style from the target domain. Image-to-image translation may be performed using a model trained with machine learning. However, when translating images from a source domain to a target domain, these models are trained to match the target domain distribution, where they may hallucinate images by adding or removing image features. This may cause issues when the target distribution during training has over or under representation of known or unknown labels compared to the test time distribution. For example, if a target domain includes only healthy tissue, the style transfer may incorrectly remove unhealthy tissues from the source domain.

In recent years, generative networks such as generative adversarial networks (GANs) have been used for producing visually compelling synthetic patient images using image to image translation. However similar to previous models, when these networks are exclusively trained on real patient images, multiple challenges arise. First, there is a lack of quantitative ground truth. In the context of attributes such as size, concentration, and location, this ground truth is crucial but unavailable which results in inadequacy for quantitative assessments and optimizations - which highlights the importance of VCT methods in the first place. Second, there may be drift from the original. There is a potential for the synthetic images to drift away from the unknown original ground truth. Given that there is not a clear benchmark (the ground truth) to refer to, rectifying any such deviations becomes a challenging task. This ambiguity may be particularly detrimental in applications centered on post-processing optimization.

Embodiments described herein provide unpaired Image-to-Image translation that leverages advanced machine learning architectures that are configured to use specialized loss functions in order to maintain ground truth values of the source domain. In an example, a GAN architecture uses two primary AI architectures: a generator, which generates images, and a discriminator, which evaluates them. The generator attempts to produce images that the discriminator cannot differentiate from real ones, fostering a continuous cycle of refinement and improvement. To enhance the realism of computational model-based medical images, the disclosed methods and system utilizes images from physical phantoms (e.g., 3D-printed constructs) or simulated images (such as XCAT) as the primary input. Real patient images serve as the secondary input. The objective is to train the model to transform the primary input into the secondary input (resembling real patient CT scans) while retaining the original's voxel properties.

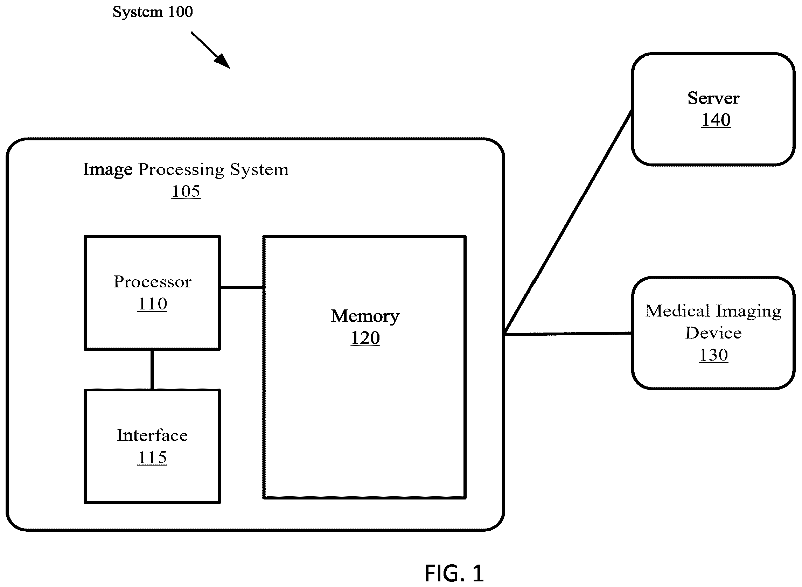

Figure 1 depicts an example system 100 for training and implementing a network for unpaired Image-to-Image translation that maintains ground truth. Figure 1 includes an image processing system 105, a server 140, and a medical imaging device 130. Fewer or more devices may be included or excluded. For example, the medical imaging device 130 may not be used if real patient images are acquired from other sources, such as a medical imaging database. The image processing system 105 is configured to generate an image / digital twin from a phantom and generate, using an image to image model, a ground truth preserving image that includes the realism and variability observed in real patient scans. The medical imaging device 130 may be used to acquire real patient images for training the model. The server 140 may provide processing, storage, and an interface for the implementation of the systems and methods as described herein. In an example, an XCAT image is derived from a digital phantom. Real patient image(s) are used that may be acquired using a medical imaging device 130. The XCAT images and real patient image(s) are used to train a network to generate a ground truth preserving image that is generated using an unpaired Image-to-Image translation as described herein. The ground truth preserving image maintains the information of the image of the XCAT image while being depicted with the style and realism of the real patient image(s).

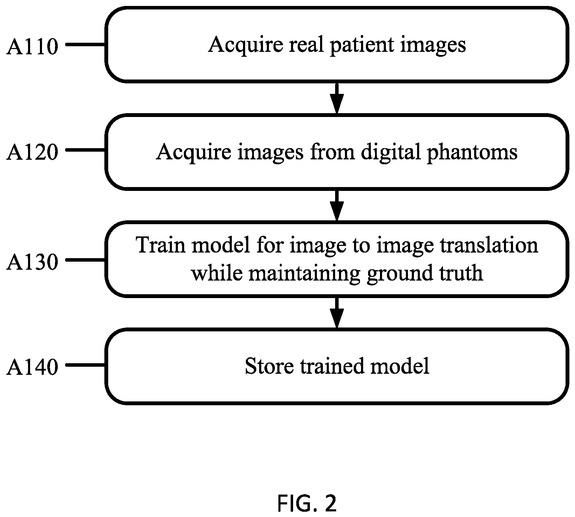

Figure 2 depicts an example method for training a network for unpaired Image-to-Image translation that maintains ground truth. The acts are performed by the system of Figures 1, 3-7, other systems, a workstation, a computer, and/or a server. Additional, different, or fewer acts may be provided. In an embodiment, using advanced generator architectures simulated images are the primary input to the unpaired Image-to-Image translation network, with real patient images acting as the secondary input.

Any type of machine learning architecture may be used for the unpaired Image-to-Image translation network. Example architectures may be based on generator type architectures such as generative AI or adversarial (GAN) type architectures, among others. Variational autoencoders (VAEs) are generative models that learn the underlying probability distribution of a dataset and generate new samples using an encoder-decoder architecture. Auto-regressive models are statistical models used to predict future values based on past values. Stable Diffusion is an AI model for creating AI images through the Forward Diffusion and Reverse Diffusion Processes. Transformers are a neural network that use an encoder-decoder structure to generate an output. GAN architectures use a generator that is tasked with converting the simulated images into realistic CT-like visuals, and a discriminator that evaluates and critiques these transformations. The iterative fight (hence adversarial) between the two networks refines the image quality, with the goal to maintain the original ground truth while approaching the realism of patient scans. To ensure ground truth preservations, specialized loss functions are integrated into the training to ensure that the generated images retain, for example, the original HU values. Additionally, or alternatively, other techniques may be used such as using a HU Value Distribution Loss, comparing HU value histograms between original and generated images, minimizing differences with methods like Earth Mover's Distance, calculating a ROI loss, a gradient penalty, a feature matching loss, using a conditional GAN, enforcing a physical simulation consistency, medical regularization, among other methods.

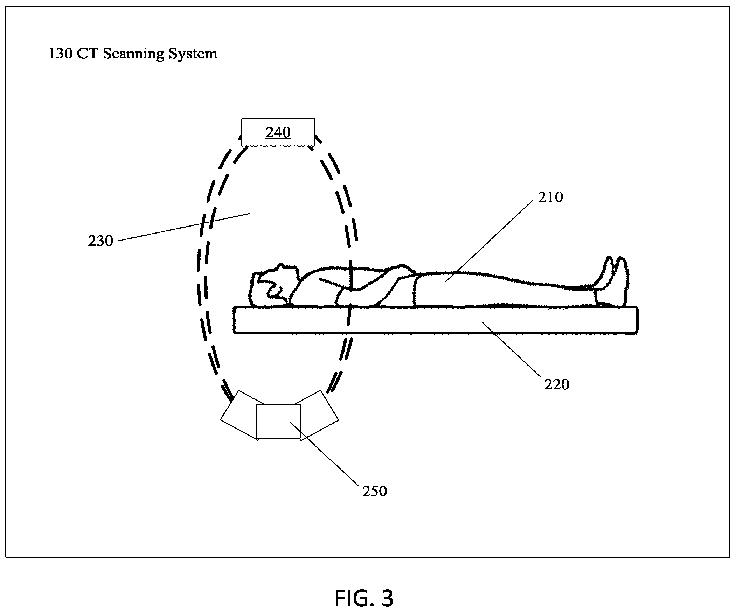

At act A110 a plurality of real patient images are acquired, for example by the medical imaging device or from a medical image database. In an example, a medical imaging device performs computed tomography (CT) to produce the real patient images of one or more real patients. While the examples describe below are described with respect to CT imaging, other types of scanners may be used (for example, MR, PET, SPECT, or other medical imaging devices). Figure 3 depicts an example CT imaging system 100. The CT scanning device as depicted is only exemplary, and a variety of CT scanning systems can be used to collect the CT data. An object 110 (e.g., a patient 110) is positioned on a table 120 that is configured, via a motorized system, to move the table 120 to multiple positions through a circular opening 130 in the CT imaging system 100. An X-ray source 140 (or other radiation source) and detector element(s) 150 are a part of the CT imaging system 100 and are configured to rotate around the subject 110 on a gantry while the subject is inside the opening / bore 130. The rotation may be combined with movement of the bed to scan along a longitudinal extent of the patient 110. Alternatively, the gantry moves the source 140 and detector 150 in a helical path about the patient 110. In the CT imaging system 100, a single rotation may take approximately one second or less. During the rotation of the X-ray source 140 and/or detector, the X-ray source 140 produces a narrow, fan-shaped (or cone-shaped) beam of X-rays that pass through a targeted section of the body of the subject 110 being imaged. The detector element(s) 150 (e.g., multi-ring detector elements) are opposite the X-ray source 140 and register the X-rays that pass through the body of the subject being imaged and, in that process, record a snapshot used to create an image. Many different snapshots at many angles through the subject are collected through one or more rotations of the X-ray source 140 and/or detector element(s) 150. The image data generated by the collected snapshots are transmitted to a control unit that stores or processes the image data based on the snapshots into one or several cross-sectional images or volumes of an interior of the body (e.g., internal organs or tissues) of the subject being scanned by the CT imaging system 100. Any now known or later developed CT system may be used. Other x-ray scanners, such as a CT-like C-arm scanner, may be used.

The medical imaging device 130 is configured to generate imaging data or medical images of a patient 110. The imaging data or the medical image is data representing a two-dimensional slice or a three-dimensional volume of the subject. The data may be in any format. The three-dimensional representation may be formatted as a stack or plurality of two-dimensional planes or slices. Values are provided for each of multiple locations distributed in two or three dimensions. The medical imaging data is acquired as one or more frames of data. The frame of data represents the scan region at a given time or period. The dataset may represent the area or volume over time, such as providing a 4D representation of the subject. While the terms image and imaging are used, the image or imaging data may be in a format prior to actual display of the image. For example, the medical imaging data may be a plurality of scalar values representing different locations in a Cartesian or polar coordinate format different than a display format. As another example, the medical image may be a plurality of red, green, blue (e.g., RGB) values output to a display for generating the image in the display format. The medical image may be currently or previously displayed image in the display or another format. The imaging data is a dataset that may be used for imaging, such as scan data or a generated image representing a portion of the patient.

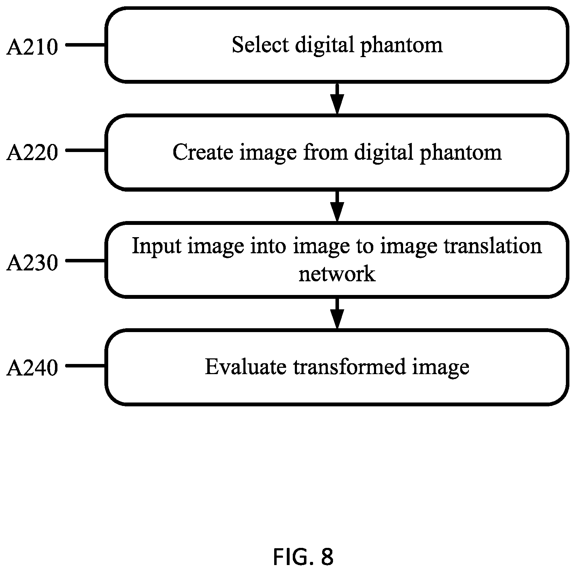

The medical imaging data or medical image is processed by the image processing system 105. The image processing system 105 includes a processor 110, interface 115, and memory 120. The image processing system 105 may receive or transmit data to and from the server 140 that may also be configured to process the image or store data for future image processing or training / storage of machine trained models. The image processing system 105 is configured to train and implement the network for unpaired Image-to-Image translation that maintains ground truth. The processor 110 is a general processor, digital signal processor, graphics processing unit, application specific integrated circuit, field programmable gate array, artificial intelligence processor, digital circuit, analog circuit, combinations thereof, or other now known or later developed device for processing images, training a model, image to image translation, etc., among other steps described below. The processor is a single device, a plurality of devices, or a network. For more than one device, parallel or sequential division of processing may be used. Different devices making up the processor 110 may perform different functions. In one embodiment, the processor 110 is a control processor or other processor of the medical imaging device 130. In other embodiments, the processor 110 is part of a separate workstation or computer. The processor 110 operates pursuant to stored instructions to perform various acts described herein. The processor 110 is configured by software, design, firmware, and/or hardware to perform any or all of the acts of Figures 2 and 8. In an embodiment, the real patient images may be acquired at a previous time and stored in, for example, a database. The method may acquire the real patient images from the database.

At act A120, a plurality of images of virtual digital phantoms and/or physical phantoms are acquired. Different types of phantoms may be used depending on the intended use. Physical, for example 3D printed phantoms may be used with a scanning device to create digital twins and/or virtual images. Digital phantoms may be scanned using a simulator. For example, there are several different types of computational digital phantoms: (a) mathematical phantoms based on equations or geometric primitives, (b) voxelized phantoms based on segmented imaging data, and (c) BREP phantoms based on segmented data but fitting high-level surfaces to the structures. Mathematical phantoms use equations or simple geometric primitives to define the organs and structures in the body. Voxelized phantoms use 3-D cuboids or voxels to define the anatomical structures based on the segmentation of patient medical images. BREP phantoms combine the advantages of voxelized and mathematical models. Based on segmented patient data, BREP phantoms use advanced surface representations such as nonuniform rational b-splines or polygon meshes to define each organ or structure. The advanced surfaces can realistically model the anatomy while providing a mathematical basis to simulate anatomical changes or motion. Computational models such as extended cardiac-torso (XCAT) phantoms provide excellent ground truth for many different clinical tasks. Since the exact anatomy and physiology of the phantoms are known, the images provide a gold standard for analysis and evaluation.

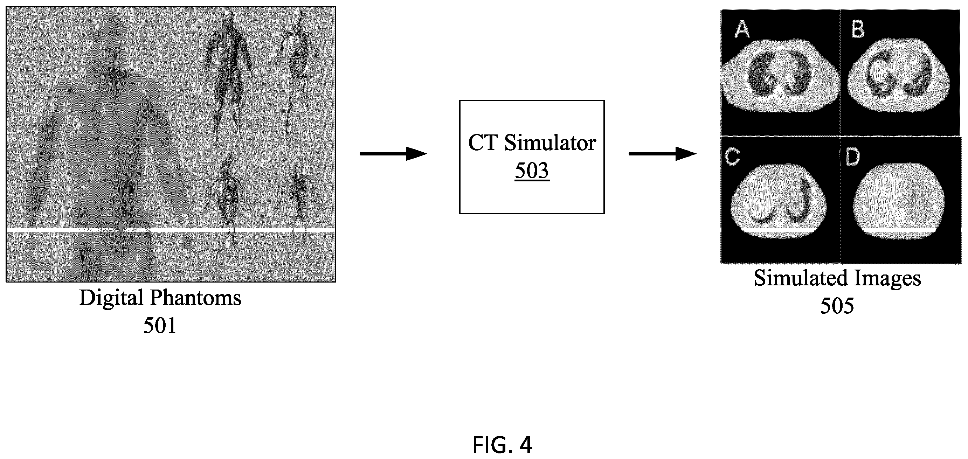

Figure 4 depicts one system for acquiring images of phantoms. Figure 4 depicts several views of an XCAT phantom 501 of a patient. In an embodiment, XCAT phantoms 501 are used to provide the computational models from which the images 505 are generated by the CT simulator 503. XCAT phantoms 501 may be based on human imaging data and include detailed whole-body models for different genders / body types / etc. Thousands of anatomical structures may be defined in the models. Combined with freely available models of the imaging process, the phantoms are capable of simulating realistic imaging data, including various artifacts of current scanners. When create, tissue characteristics are assigned to the different tissues, such as attenuation for CT, uptake for nuclear medicine, T1, T2, and spin density for MRI or acoustic properties for ultrasound. Based on these characteristics and other parameters, imaging simulators 503 input the phantom 501 and generate the simulated imaging data 505. In one example, a CT simulator 503 is used to generate images from the XCAT models 501. The image data 505 is unpaired with the real patient images acquired at act A110; however, it may represent the same organ / view / parameters as the real imaging data. The CT simulator 503 uses, for example, Monte Carlo simulations or ray-tracing to simulate a CT scanner 130.

At act A130, a model is trained to transform the plurality of images of virtual digital phantoms to resemble the plurality of real patient images, wherein the model is trained using at least one specialized loss function that ensures the transformed images retains original ground truth values of the virtual images. The model is a model for unpaired image to image translation. Unpaired image to image translation attempts aims to learn mappings that can map images from one domain to another domain while preserving the content of the input image. For example, the network is trained to translate an image from a source domain X to a target domain Y in the absence of paired examples. The goal is to learn a mapping G:X→Y such that the distribution of images from G(X) is indistinguishable from the distribution Y using an adversarial loss.

In an embodiment, the machine learned network(s) or model(s) include a neural network that is defined as a plurality of sequential feature units or layers. Sequential is used to indicate the general flow of output feature values from one layer to input to a next layer. Sequential is used to indicate the general flow of output feature values from one layer to input to a next layer. The information from the next layer is fed to a next layer, and so on until the final output. The layers may only feed forward or may be bi-directional, including some feedback to a previous layer. The nodes of each layer or unit may connect with all or only a sub-set of nodes of a previous and/or subsequent layer or unit. Skip connections may be used, such as a layer outputting to the sequentially next layer as well as other layers. Rather than pre-programming the features and trying to relate the features to attributes, the deep architecture is defined to learn the features at different levels of abstraction based on the input data. The features are learned to reconstruct lower-level features (i.e., features at a more abstract or compressed level). Each node of the unit represents a feature. Different units are provided for learning different features. Various units or layers may be used, such as convolutional, pooling (e.g., max pooling), deconvolutional, fully connected, or other types of layers. Within a unit or layer, any number of nodes is provided. For example, 100 nodes are provided. Later or subsequent units may have more, fewer, or the same number of nodes.

For training and applying a machine trained model there are two stages, a training stage for generating or training the model using a collection of training data and an application stage for applying the generated / trained entity matching network to new unseen (unlabeled) data. The training stage includes acquiring training data, processing the training data, and inputting the training data into the model in order to generate a trained model. The output is a trained model that is applied in the application stage. The application stage includes receiving new data and applying the trained model that was trained during the training stage to output a transformed image. The training stage may be performed at any point prior to the application stage. The training stage may be repeated after new training data is acquired. The application stage may be performed at any point after the training stage generates the trained network and new data is received.

The model is trained using machine learning. In an embodiment, the model is a neural network that is iteratively trained to perform style transfer from a first domain to a second domain while maintaining ground truth values. The network may be specifically configured for each respective task. Different networks and configurations may be used. For example, a DenseNet or other network arrangements may also be used for the trained networks or other trained networks described above for segmentation, classification, or analysis. A DenseNet connects each layer in a network to every other layer in a feed-forward fashion. For each layer in the DenseNet, the feature-maps of all preceding layers are used as inputs, and the output feature-map of that layer is used as input into all subsequent layers. In the DenseNet, for each layer, the feature maps of all preceding layers are used as inputs, and its own feature maps are used as inputs into all subsequent layers. To reduce the size of the network, the DenseNet may include transition layers. The layers include convolution followed by average pooling. The transition layers reduce height and width dimensions but leave the feature dimension the same. The neural network may further be configured as a U-net. The U-Net is an autoencoder in which the outputs from the encoder-half of the network are concatenated with the mirrored counterparts in the decoder-half of the network. Skip connections prevent the middle of the network from becoming a bottleneck.

Other deep architectures that may be used include convolutional neural network (CNN) or deep belief nets (DBN), but other deep networks may be used. CNN learns feed-forward mapping functions while DBN learns a generative model of data. In addition, CNN uses shared weights for all local regions while DBN is a fully connected network (e.g., including different weights for all regions of an image). The training of CNN is entirely discriminative through back-propagation. DBN, on the other hand, employs the layer-wise unsupervised training (e.g., pre-training) followed by the discriminative refinement with back-propagation if necessary. In an embodiment, the arrangement of the trained network is a fully convolutional network (FCN). Alternative network arrangements may be used, for example, a 3D Very Deep Convolutional Networks (3D-VGGNet). VGGNet stacks many layer blocks containing narrow convolutional layers followed by max pooling layers. A 3D Deep Residual Networks (3D-ResNet) architecture may be used. A Resnet uses residual blocks and skip connections to learn residual mapping.

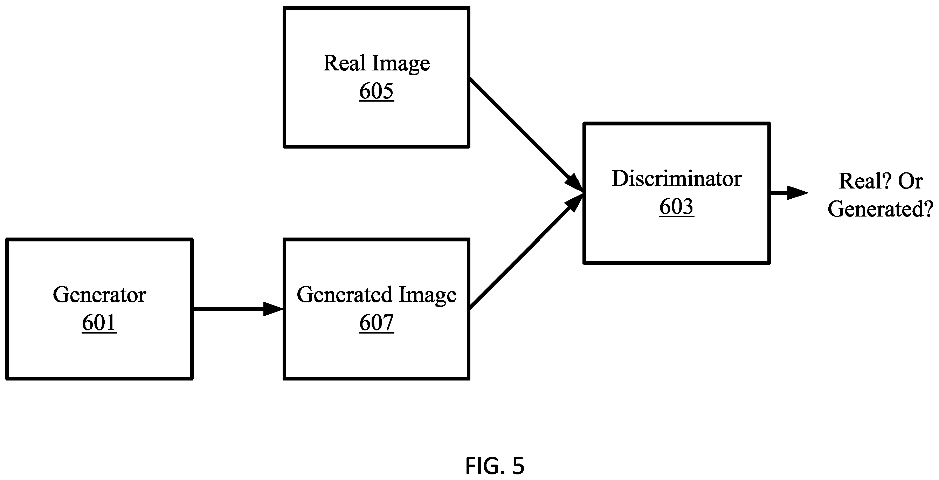

In an embodiment, the model is trained using adversarial learning. Figure 5 depicts an example of a GAN. Adversarial learning uses at least two networks, a generator 601 and a discriminator 603. The generator 601 generates data 607 which the discriminator 603 attempts to distinguish from "real" data 605. One or more of the networks may be adjusted iteratively until the generator 601 becomes "good" enough to fool the discriminator 603.

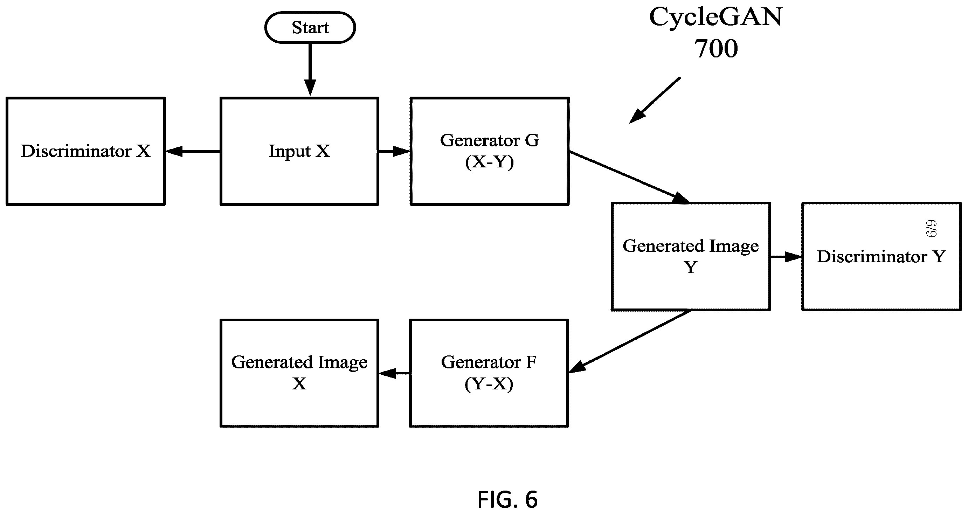

In an embodiment, the model is configured as a Cycle-Consistent Adversarial Network, also referred to a CycleGAN. Figure 6 depicts an example of a CycleGAN. In Figure 6, there are two generators G and F and two discriminators X and Y. The generator G translates the distribution from one style X to another style Y, so that the discriminator Y cannot distinguish the transformed style Y=G(X) from the original style Y. One problems with this system is that the generator may translate all the images in the same way, so it can only generate a single example Y, which is not the desired task. To alleviate this problem, the CycleGAN structure uses a constraint by defining another generator F whose role is to be the inverse transform of G. This guarantees that the transformation of X will not be reduced to a single example. A new loss function characterizing cycle consistency loss is added during training, encouraging transformations to verify the properties FGX≈X and that G(FY)≈Y. There are two discriminators: Discriminator Y that discriminates generated Y from Y and Discriminator X which discriminates the generated X from real X. The cycle-consistency loss is separated into two distinct pieces, the first (1) corresponding to the loss between the elements of X and their reconstructions and the second (2) corresponding to the loss between the elements of Y and their reconstructions. During training, the generators and discriminators optimize the same general loss function, which is made up of two loss sub-functions associated with the generators (adversarial loss): which will be maximized by the discriminators (so that they can distinguish generation from reality) and minimized by the generators, so that they can create examples that are increasingly indistinguishable from real data.

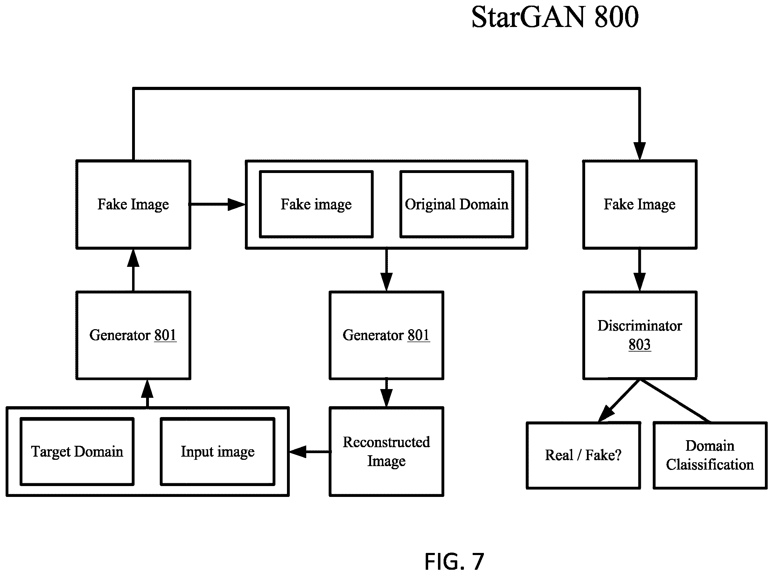

In another embodiment, the model is configured using a StarGAN architecture. Figure 7 depicts an example of a StarGAN architecture. StarGAN includes two modules, a discriminator 803 and a generator 801. The discriminator 803 learns to distinguish between real and fake images and classify the real images to its corresponding domain. The generator 801 takes in as input both the image and target domain label and generates a fake image. The target domain label is spatially replicated and concatenated with the input image. The generator 801 tries to reconstruct the original image from the fake image given the original domain label. The generator 801 tries to generate images indistinguishable from real images and classifiable as target domain by the discriminator 803. The discriminator 803 attempts to distinguish it inputs and further classifies an image to its corresponding domain, so that the generator 801 tries to generate images that are indistinguishable from real images and are classifiable as target domain by discriminator 803. i.e., the generator 801 will ultimately learn to generate realistic images corresponding to the given target domain. The discriminator 803 has two things to do. First the discriminator 803 attempts identify whether an image is fake or not. Second, with the help of an auxiliary classifier, the discriminator 803 also predicts the domain of the image given as input to discriminator 803. The generator 801 weights are adjusted so that the images generated are realistic. The generator 801 weights are further adjusted so that the generated images are classifiable as target domain by the discriminator. Finally, the generator 801 weights are adjusted based on the additional loss value / function as described below. The generator 801 tries to reconstruct the original image from the fake image given the original domain label. The single generator 801 is used twice, first to translate an original image into an image in the target domain and then to reconstruct the original image from the translated image. Other network configurations may be used such as pix2pix, discogan, dualgan, etc. Any network architecture that provides unpaired image to image translation may be used.

During training, an additional loss value, parameter, or step is used to maintain the ground truth values of the computational phantom images. In an example, a CycleGAN as described above includes an objective function to minimize a loss. The total loss may be separated into three parts, adversarial losses, each for both domains, and cycle consistency loss. The adversarial loss function used in CycleGAN is similar to that of a typical GAN. It involves setting an objective for the generator G to produce images G(x) that are visually similar to images from domain Y, while the discriminator Dy aims to differentiate between the generated samples G(x) and real samples y. The goal is to minimize this objective for G, while the adversary D attempts to maximize it. Additionally, a similar loss function is introduced for the mapping function F: Y -> X and its discriminator Dx. For each image x from domain X, the image translation cycle should be able to bring x back to the original image (forward cycle consistency), i.e., x → G(x) → F(G(x)) ≈ x. Similarly, for each image y from domain Y , G and F should also satisfy backward cycle consistency: y → F(y) → G(F(y)) ≈ y. An additional loss value / function is be added to the adversarial losses for either of the domains. In an embodiment, a loss value is used to compare HU value histograms between original and generated images, minimizing differences with methods like Earth Mover's Distance. Hounsfield units (HU) are a dimensionless unit used in computed tomography (CT) scanning to express CT numbers in a standardized and convenient form. Hounsfield units are obtained from a linear transformation of the measured attenuation coefficients. This transformation is based on the radiodensities of air and pure water (radiodensity of distilled water = 0HU, radiodensity of air = 1000HU). The linear transformation produces a Hounsfield scale that displays as gray tones. More dense tissue, with greater X-ray beam absorption, has positive values and appears bright; less dense tissue, with less X-ray beam absorption, has negative values and appears dark. The use of the HU to measure tissue density has aided radiologists in the interpretation of images and diagnosis of disease. Maintaining the HU values between the source domain and the target domain in the image to image translation would maintain the underlying ground truth features of the source domain and thus allow for better analysis and evaluation of generated images. In another embodiment, a region of interest (ROI loss) is used. For areas with for example, known iodine concentrations, a Mean Squared Error is used to match HU values between original and generated images and incorporate that info in the cost function loop feedback. In another embodiment, a gradient penalty is used. The gradient penalty provides consistent HU value transitions at tissue boundaries in both image types. In another embodiment, a feature matching loss is used. A pre-trained CT model may be used to compare deep features, focusing on HU values, between images.

In another embodiment, a conditional GAN is used that uses HU metrics to create consistent images. With a conditional GAN, class labels based on HU metrics are input in the generator and discriminator to guide the image generate. In an example, the labels guide the generator's production to generate more specific information. For example, directing the generator to generate a specific tissue, artifact, etc. Other losses may be used such as to ensure physical simulation consistency or medical regularization. The loss function may penalize deviations from X-ray attenuation simulations in generated images or penalize generated images that don't meet known HU thresholds for specific materials respectively. In another step, radiologist reviews may be used to refine generated image accuracy. The use of the additional loss functions or steps provides that the ground truth of the source domain is preserved.

In an embodiment, known details for pathologies or physical features with exact sizes, concentrations, and material attributes are added into normal scans. Pathologies may include but not limited to: Aortic plaques, Tumors (both benign and malignant), Pulmonary nodules and lung cancers, Brain abnormalities (e.g., hemorrhage, infarction, aneurysms), Liver lesions (such as cysts, hemangiomas, hepatomas), Kidney stones, Aortic aneurysms, Pneumonia and other lung infections, Chronic obstructive pulmonary diseases (COPD), and/or Pulmonary embolism. Because the exact sizes, concentrations, and material attributes are maintained in the process of generating the realistic images, this embodiment is particularly valuable for training machine learning models, as it offers a diverse set of positive samples while keeping the ground truth accurate.

Once trained, at act A140, the image to image network is stored. The network may be used to provide realistic images for different application such as in virtual clinical trials or for digital twins. The digital twin is a virtual model of a physical object, for example a 3D printed anatomy. The digital twin may be linked to the physical object or may be static.

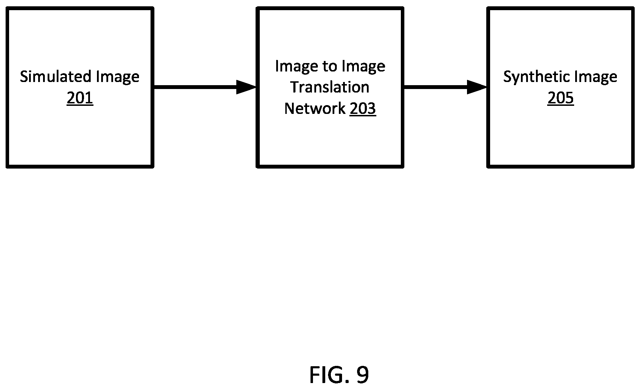

Figure 8 depicts an example method for generating a synthetic image / digital twin, for example when performing a virtual clinical trial. The acts are performed by the system of Figures 1, 3-7, other systems, a workstation, a computer, and/or a server. Additional, different, or fewer acts may be provided. Figure 9 depicts an example workflow for generating the synthetic image. In Figure 9, the simulated image 201 is input into the image to image translation network 203 which outputs the synthetic (transformed) image 205.

In Figure 8, at act A210, a digital phantoms is selected. A user may select, for example, the type of body, gender, etc. for a digital phantom. In an embodiment, generative AI is used to generate the image. A text-to-image model may be used to provide a realistic image when prompted with text details for the typo of phantom, type of scan, etc. A user opens a chatbot and asks the system to create a medical image. The user is prompted (or provides) the type of digital phantom, the parameters for the medical imaging simulator, and any other variables. The chatbot uses the entered information to select a phantom, a simulator, and an image to image network, which it uses to generate the requested image. Alternatively, a digital twin may be selected by the user. The digital twin is a digital representation of a physical object.

At act A220, a simulated image 201 is created from the digital phantom / digital twin using a medical imaging simulator. Any medical imaging simulator may be used. The user may be prompted to select what type of imaging modality that is to be simulated. The user may have the option to select different sequences or parameters of the simulated scan. The output is an image that include ground truth values for one or more features based on which digital phantom was selected in act A210.

At act A230, the image is input into an image to image translation network 203 configured to maintain ground truth values for the image while transforming the image to look realistic e.g., similar to real medical images. The image to image translation network is configured to transfer a style from a second domain to the image. Any image to image translation network may be used. In one example, the image to image translation is performed using a CycleGAN, or Cycle-Consistent Generative Adversarial Networks. The CycleGAN learns to convert an image from X into Y, as well as an image from Y into X, without any specific order or pairing between the images in the two collections. The objective of the training process is to develop a generator G: X -> Y that produces a distribution of images from G(X) that are similar enough to the distribution Y, so that they cannot be distinguished from the real images using an adversarial loss. However, since this mapping can be difficult to constrain, CycleGAN also introduces an inverse mapping F: Y -> X. The reason for F is to include a cycle consistency loss to enforce that F(G(X)) and X (and vice versa) are approximately equal.

One or more additional loss values are used by the image to image translation network. For example, in an embodiment, a dataset of XCAT model images with ground truth HU values and a separate set of real patient CT images are used to train the image to image translation network. The GAN is trained to bridge the gap between XCAT and real images, ensuring the transformed HU values closely align with the original, thereby creating a realistic image while maintaining accurate ground truth.

The output of the image to image translation network is an image that appears realistic (has the style of a real image) while still including the ground truth of the digital phantom.

At act A240, the synthetic image 205 is evaluated. In the real domain, this evaluation may be performed by an expert imaging physician (for example a radiologist). In the virtual domain, the physician may be replaced by a virtual observer, with its performance aspired to match that of a real human expert, just like virtual patients and virtual imaging systems aim to emulate their corresponding real counterparts as closely as possible. For example, in simulated CT imaging, a broad range of VCT studies have been conducted with more focus on dosimetry and image quality assessments. With CT being the single largest source of medical radiation exposure, reducing the dose to patients without sacrificing image quality is desired. Dose can be studied using VCTs in which computational phantoms are "imaged" using MC-based CT simulators. Studies of this nature cannot be performed using live subjects due to ethical concerns. Organ doses may be estimated under various imaging protocols across virtual populations of adults, pediatrics, and pregnant patients. In addition, these VCTs may be used to investigate the relationship between the estimated organ doses and CT parameters and patient attributes.

The output of the processes and methods may be output for further processing or displayed to an operator. The image processing system 105 includes an operator interface 15, formed by an input and an output. The input may be an interface, such as interfacing with a computer network, memory, database, medical image storage, or other source of input data. The input may be a user input device, such as a mouse, trackpad, keyboard, roller ball, touch pad, touch screen, or another apparatus for receiving user input. The input may receive a scan protocol, imaging protocol, or scan parameters. An individual may select the input, such as manually or physically entering a value. Previously used values or parameters may be input from the interface. Default, institution, facility, or group set levels may be input, such as from memory to the interface.

The output may be a display device or any other type of interface. The images, for example, as output by the method are displayed. For example, an image of a region of the patient is displayed. A generated image for a selected model and simulated scan is presented on a display of the operator interface 115. An analysis / interpretation may also be displayed on the display device. The image processing system 105 may be configured to generate a report / evaluation for the image that is displayed on the display device. The display is a CRT, LCD, plasma, projector, printer, or other display device. The display is configured by loading an image to a display plane or buffer. The display is configured to display the reconstructed MR image of the region of the patient. The operator interface may include form a graphical user interface (GUI) enabling user interaction with the image processing system 105 and enables user modification in substantially real time.

While the invention has been described above by reference to various embodiments, it should be understood that many changes and modifications can be made without departing from the scope of the invention. It is therefore intended that the foregoing detailed description be regarded as illustrative rather than limiting, and that it be understood that it is the following claims, including all equivalents, that are intended to define the scope of this invention.

## Claims

1. A method for training a model for unpaired Image-to-Image translation of medical images, the method comprising:
acquiring (A110) a plurality of real patient images;
acquiring (A120) a plurality of synthetic images (505) of digital phantoms (501); and
training (A130) a model to transform the plurality of synthetic images (505) of digital phantoms (501) to resemble the plurality of real patient images (605), wherein the model is trained using at least one specialized loss function that ensures the transformed images retain original ground truth values of the plurality of synthetic images (505);
**characterized in that** the specialized loss function comprises a loss value based on a comparison of Hounsfield unit (HU) value histograms between original and generated images.

2. The method of claim 1, wherein the plurality of real patient images (605) are provided by scanning a patient (210) using a CT medical imaging system (130).

3. The method of claim 1 or 2, wherein the plurality of synthetic images (505) are provided by scanning the digital phantoms (501) using a medical imaging system simulator (503).

4. The method of any one of claims 1 - 3, wherein the model comprises a generator network (601) trained using an adversarial machine learning process.

5. The method of claim 4, wherein the generator network (601) is trained using a CycleGAN architecture (700) and/or using a STARGAN architecture (800).

6. The method of any one of claims 1 - 5, wherein the specialized loss function further comprises at least one of:
calculating a region of interest loss,
a feature matching loss,
a loss that enforces regularization or a physical simulation consistency,
a comparison between the output image and an annotated image.

7. A system (100), in particular for performing a virtual clinical trial, the system (100) comprising:
a plurality of virtual digital phantoms (501) and/or physical phantoms, wherein each of the virtual digital phantoms (501) and/or physical phantoms includes one or more ground truth values for a feature included in the virtual digital phantoms (501) and/or physical phantoms;
a virtual imaging simulator configured to generate a virtual image (505) from each of the plurality of virtual digital phantoms (501) and/or physical phantoms; and
a model configured for unpaired Image-to-Image translation, the model configured to transform the virtual images (505) to resemble real patient images (605) while maintaining the one or more ground truth values, wherein the model is trained according to the method of any one of claims 1 - 6.

8. The system (100) of claim 7, wherein the plurality of virtual digital phantoms (501) are XCAT models.

9. The system (100) of claim 7 or 8, wherein the virtual imaging simulator is configured to simulate a CT scan of the plurality of virtual digital phantoms (501) and/or physical phantoms.

10. The system (100) of any one of claims 7 - 9, wherein the model comprises a GAN based architecture and/or a Generative AI based architecture.

11. A method for generating a synthetic medical image, the method comprising:
selecting (A210) a digital phantom (501);
inputting (A220) the digital phantom (501) into a medical imaging simulator (503) configured to generate a simulated image (201) of the digital phantom (501);
inputting the simulated image (201) into an unpaired image to image translation network (203) configured to generate a realistic version of the simulated image (201); and
evaluating the realistic version of the simulated image (201), wherein the unpaired image to image translation network (203) is implemented through a trained model according to the method of any one of claims 1 - 6 or a system (100) according to any one of claims 7 - 10.

12. The method of claim 11, wherein the digital phantom (201), medical imaging simulator (503), and scan parameters are selected or provided by a chatbot.

## Patentansprüche

1. Verfahren zum Trainieren eines Modells für die ungepaarte Bild-zu-Bild-Übersetzung von medizinischen Bildern, wobei das Verfahren umfasst:
Erfassen (A110) von mehreren echten Patientenbildern;
Erfassen (A120) von mehreren synthetischen Bildern (505) von digitalen Phantomen (501); und
Trainieren (A130) eines Modells, um die mehreren synthetischen Bilder (505) von digitalen Phantomen (501) so umzuwandeln, dass sie den mehreren echten Patientenbildern (605) ähneln, wobei das Modell unter Verwendung mindestens einer spezialisierten Verlustfunktion trainiert wird, die sicherstellt, dass die umgewandelten Bilder ursprüngliche Grundwahrheitswerte der mehreren synthetischen Bilder (505) beibehalten;
**dadurch gekennzeichnet, dass** die spezialisierte Verlustfunktion einen Verlustwert umfasst, der auf einem Vergleich von Hounsfield-Einheit (HU) - Wert-Histogrammen zwischen originalen und erzeugten Bildern basiert.

2. Verfahren nach Anspruch 1, wobei die mehreren echten Patientenbilder (605) durch Scannen eines Patienten (210) unter Verwendung eines medizinischen CT-Bildgebungssystems (130) bereitgestellt werden.

3. Verfahren nach Anspruch 1 oder 2, wobei die mehreren synthetischen Bilder (505) durch Scannen der digitalen Phantome (501) unter Verwendung eines Simulators für medizinische Bildgebungssysteme (503) erzeugt werden.

4. Verfahren nach einem der Ansprüche 1 - 3, wobei das Modell ein Generatornetz (601) umfasst, das mit einem adversariellen Maschinenlernprozess trainiert wurde.

5. Verfahren nach Anspruch 4, wobei das Generatornetz (601) unter Verwendung einer CycleGAN-Architektur (700) und/oder unter Verwendung einer STARGAN-Architektur (800) trainiert wird.

6. Verfahren nach einem der Ansprüche 1 - 5, wobei die spezialisierte Verlustfunktion ferner mindestens eines der folgenden umfasst:
Berechnen eines Verlusts eines Bereichs von Interesse,
einen Merkmalsabgleichverlust,
einen Verlust, der eine Regularisierung oder eine physische Simulationskonsistenz erzwingt,
einen Vergleich zwischen dem Ausgabebild und einem annotierten Bild.

7. System (100), insbesondere zur Durchführung einer virtuellen klinischen Studie, wobei das System (100) umfasst:
mehrere virtuelle digitale Phantome (501) und/oder physische Phantome, wobei jedes der virtuellen digitalen Phantome (501) und/oder physischen Phantome einen oder mehrere Grundwahrheitswerte für ein in den virtuellen digitalen Phantomen (501) und/oder physischen Phantomen enthaltenes Merkmal aufweist;
einen virtuellen Bildgebungssimulator, der dafür ausgelegt ist, ein virtuelles Bild (505) von jedem der mehreren virtuellen digitalen Phantome (501) und/oder physischen Phantome zu erzeugen; und
ein Modell, das für eine ungepaarte Bild-zu-Bild-Übersetzung ausgelegt ist, wobei das Modell dafür ausgelegt ist, die virtuellen Bilder (505) so umzuwandeln, dass sie echten Patientenbildern (605) ähneln, während die ein oder mehreren Grundwahrheitswerte erhalten bleiben, wobei das Modell gemäß dem Verfahren nach einem der Ansprüche 1 - 6 trainiert wird.

8. System (100) nach Anspruch 7, wobei die mehreren virtuellen digitalen Phantome (501) XCAT-Modelle sind.

9. Das System (100) nach Anspruch 7 oder 8, wobei der virtuelle Bildgebungssimulator dafür ausgelegt ist, einen CT-Scan der mehreren virtuellen digitalen Phantome (501) und/oder physischen Phantome zu simulieren.

10. System (100) nach einem der Ansprüche 7 - 9, wobei das Modell eine GAN-basierte Architektur und/oder eine generative KI-basierte Architektur umfasst.

11. Verfahren zum Erzeugen eines synthetischen medizinischen Bildes, wobei das Verfahren umfasst:
Auswählen (A210) eines digitalen Phantoms (501);
Eingeben (A220) des digitalen Phantoms (501) in einen medizinischen Bildgebungssimulator (503), der dafür ausgelegt ist, ein simuliertes Bild (201) des digitalen Phantoms (501) zu erzeugen;
Eingeben des simulierten Bildes (201) in ein Netz für ungepaarte Bild-zu-Bild-Übersetzung (203), das dafür ausgelegt ist, eine realistische Version des simulierten Bildes (201) zu erzeugen; und
Auswerten der realistischen Version des simulierten Bildes (201), wobei das Netz für ungepaarte Bild-zu-Bild-Übersetzung (203) durch ein trainiertes Modell gemäß dem Verfahren nach einem der Ansprüche 1 - 6 oder ein System (100) gemäß einem der Ansprüche 7 - 10 implementiert wird.

12. Verfahren nach Anspruch 11, wobei das digitale Phantom (201), der medizinische Bildgebungssimulator (503) und die Scanparameter von einem Chatbot ausgewählt oder bereitgestellt werden.

## Revendications

1. Procédé d'apprentissage d'un modèle de translation d'image en image non appariée d'images médicales, le procédé comprenant :
l'acquisition (A110) d'une pluralité d'images réelles de patient ;
l'acquisition (A120) d'une pluralité d'images synthétiques (505) de fantômes numériques (501) ; et
l'entraînement (A130) d'un modèle pour transformer la pluralité d'images synthétiques (505) de fantômes numériques (501) pour ressembler à la pluralité d'images réelles de patient (605), le modèle étant entraîné à l'aide d'au moins une fonction de perte spécialisée qui garantit que les images transformées conservent les valeurs de réalité de terrain originales de la pluralité d'images synthétiques (505) ;
**caractérisé en ce que** la fonction de perte spécialisée comprend une valeur de perte basée sur une comparaison d'histogrammes de valeur d'unité Hounsfield (HU) entre les images originales et générées.

2. Procédé selon la revendication 1, la pluralité d'images réelles de patient (605) étant fournies par balayage d'un patient (210) à l'aide d'un système d'imagerie médicale CT (130).

3. Procédé selon la revendication 1 ou 2, la pluralité d'images synthétiques (505) étant fournies en balayant les fantômes numériques (501) à l'aide d'un simulateur de système d'imagerie médicale (503).

4. Procédé selon l'une quelconque des revendications 1 à 3, le modèle comprenant un réseau générateur (601) entraîné à l'aide d'un processus d'apprentissage automatique antagoniste.

5. Procédé selon la revendication 4, le réseau générateur (601) étant entraîné à l'aide d'une architecture CycleGAN (700) et/ou à l'aide d'une architecture STARGAN (800).

6. Procédé selon l'une quelconque des revendications 1 à 5, la fonction de perte spécialisée comprenant en outre au moins l'une parmi :
le calcul d'une perte de région d'intérêt,
une perte de correspondance de caractéristique,
une perte qui impose une régularisation ou une cohérence de simulation physique,
une comparaison entre l'image de sortie et une image annotée.

7. Système (100), notamment pour effectuer un essai clinique virtuel, le système (100) comprenant :
une pluralité de fantômes numériques virtuels (501) et/ou de fantômes physiques, chacun des fantômes numériques virtuels (501) et/ou des fantômes physiques comprenant une ou plusieurs valeurs de réalité de terrain pour une caractéristique incluse dans les fantômes numériques virtuels (501) et/ou les fantômes physiques ;
un simulateur d'imagerie virtuelle configuré pour générer une image virtuelle (505) à partir de chacun de la pluralité de fantômes numériques virtuels (501) et/ou de fantômes physiques ; et
un modèle configuré pour une translation d'image en image non appariée, le modèle étant configuré pour transformer les images virtuelles (505) pour ressembler à des images réelles de patient (605) tout en conservant la ou les valeurs de réalité de terrain, le modèle étant entraîné conformément au procédé selon l'une quelconque des revendications 1 à 6.

8. Système (100) selon la revendication 7, la pluralité de fantômes numériques virtuels (501) étant des modèles XCAT.

9. Système (100) selon la revendication 7 ou 8, le simulateur d'imagerie virtuelle étant configuré pour simuler un balayage CT de la pluralité de fantômes numériques virtuels (501) et/ou de fantômes physiques.

10. Système (100) selon l'une quelconque des revendications 7 à 9, le modèle comprenant une architecture à base de GAN et/ou une architecture à base d'IA générative.

11. Procédé pour générer une image médicale synthétique, le procédé comprenant :
la sélection (A210) d'un fantôme numérique (501) ;
la saisie (A220) du fantôme numérique (501) dans un simulateur d'imagerie médicale (503) configuré pour générer une image simulée (201) du fantôme numérique (501) ;
la saisie de l'image simulée (201) dans un réseau de translation d'image en image non appariée (203) configuré pour générer une version réaliste de l'image simulée (201) ; et
l'évaluation de la version réaliste de l'image simulée (201), le réseau de translation d'image en image non appariée (203) étant mis en œuvre par l'intermédiaire d'un modèle entraîné conformément au procédé selon l'une quelconque des revendications 1 à 6 ou d'un système (100) selon l'une quelconque des revendications 7 à 10.

12. Procédé selon la revendication 11, le fantôme numérique (201), le simulateur d'imagerie médicale (503) et les paramètres de balayage étant sélectionnés ou fournis par un chatbot.
